(19) Europäisches Patentamt
European Patent Office
Office européen des brevets

(11) Numéro de publication : 0 084 470
B1

(12) # FASCICULE DE BREVET EUROPÉEN

(45) Date de publication du fascicule du brevet : **22.10.86**

(21) Numéro de dépôt : **83400024.2**

(22) Date de dépôt : **05.01.83**

(51) Int. Cl.[4] : **C 07 C102/08**, C 07 C 99/10, C 07 C148/00, C 08 F 8/30, C 08 F 8/12

(54) **Procédé d'hydrolyse catalytique d'un alpha-amino-nitrile en phase hétérogène, ainsi que les résines polymères à acitivité catalytique pour la mise en oeuvre du procédé.**

(30) Priorité : **15.01.82 FR 8200600**

(43) Date de publication de la demande : **27.07.83 Bulletin 83/30**

(45) Mention de la délivrance du brevet : **22.10.86 Bulletin 86/43**

(84) Etats contractants désignés : **BE CH DE FR GB IT LI NL**

(56) Documents cités :
**AU-B- 458 714**
**FR-A- 2 372 797**
**FR-A- 2 396 031**
**FR-A- 2 405 924**

(73) Titulaire : **Etablissement Public dit: CENTRE NATIONAL DE LA RECHERCHE SCIENTIFIQUE (CNRS)**
**15, Quai Anatole France**
**F-75700 Paris (FR)**

(72) Inventeur : **Commeyras, Auguste**
**Impasse des Ecoles Clapiers**
**F-34170 Castelnau le Lez (FR)**
Inventeur : **Taillades, Jacques**
**9 rue des Erables Lot. du Vert Pré Clapiers**
**F-34170 Castelnau le Lez (FR)**
Inventeur : **Brugidou, Jean**
**8 rue Jacques Boe La Paillade**
**F-34100 Montpellier (FR)**
Inventeur : **Sola, Régine**
**l'Orée du bois II 171, avenue d'Occitanie**
**F-34000 Montpellier (FR)**
Inventeur : **Previero, Aldo**
**Mas de la Droletta Rue du Père Prévost**
**F-34000 Montpellier (FR)**
Inventeur : **Mion, Louis**
**477 rue d'Alco**
**F-34100 Montpellier (FR)**
Inventeur : **Pascal, Robert**
**Rés. Chene Colombière Bat. I 2 58 av. d'Occitanie**
**F-34100 Montpellier (FR)**
Inventeur : **Lasperas, Monique**
**793 Bd de la Lironde Montferrier sur Lez**
**F-34980 St Gély du Fesc (FR)**
Inventeur : **Rousset, Alain**
**Orée du Bois 171 av. d'Occitanie**
**F-34100 Montpellier (FR)**

(74) Mandataire : **Martin, Jean-Jacques et al**
**Cabinet REGIMBEAU 26, Avenue Kléber**
**F-75116 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention se rapporte à un procédé d'hydrolyse catalytique chimique d'un α-amino-nitrile ou de l'un de ses sels. Conformément au procédé de la présente invention, cette hydrolyse catalytique d'un α-amino-nitrile ou de l'un de ses sels peut conduire, en fonction des conditions réactionnelles particulières, soit à la formation d'un α-amido-amide, soit directement à la formation d'un sel d'α-amino-acide, ce dernier pouvant être facilement transformé, par simple neutralisation, en l'α-amino-acide libre correspondant. La présente invention concerne donc également de façon particulière la préparation d'α-amino-acides, sous la forme de leur mélange racémique, obtenus à partir d'α-amino-nitrile, de leurs sels ou encore à partir de leurs précurseurs.

Il convient à ce propos de rappeler que les α-amino-acides revêtent un intérêt industriel incontestable. Certains d'entre eux peuvent en effet être utilisés en médecine humaine ou vétérinaire, de même que dans l'alimentation, par exemple en vue de compléter des rations alimentaires. D'autres de ces composés peuvent également entrer par exemple dans la composition de savons ou de cosmétiques.

Dans la technique antérieure, les α-amino-acides étaient préparés à partir de leurs précurseurs aldéhydiques correspondants, par la réaction de Strecker ou encore par une des nombreuses modifications apportées à la réaction originale, et en particulier par la mise en œuvre du procédé décrit dans le brevet français n° 2 372 797. Conformément au procédé de ce brevet antérieur, l'α-amino-nitrile intermédiaire ou l'un de ses sels est hydrolysé catalytiquement en milieu basique, en faisant réagir une solution aqueuse contenant au moins un dérivé carbonylé sur ledit α-amino-nitrile ou sur l'un de ses sels, en présence d'ions hydroxydes. Un tel procédé, extrêmement sélectif et économiquement favorable, nécessite cependant la séparation du catalyseur carbonylé du milieu réactionnel et son éventuel recyclage. De plus, dans ce procédé antérieur, le choix du catalyseur carbonylé se trouve fortement limité par sa nécessaire stabilité en milieu basique homogène.

L'état de la technique peut en outre être complété par la citation de FR-A-2 396 031 et AU-B- 458 714 concernant l'utilisation de polymères en tant que catalyseur qui sont cependant utilisés dans un domaine fondamentalement différent de l'hydrolyse d'alpha-amino-nitrile, par exemple comme agent viscosant dans des compositions de traitement photographique.

La présente invention a précisément eu pour but d'écarter ces inconvénients inhérents aux procédés de la technique antérieure la plus proche évoquée précédemment.

Le procédé, conforme à la présente invention, est caractérisé en ce que l'on fait réagir en milieux aqueux ledit alpha-amino-nitrile ou l'un de ses sels, en phase hétérogène et en présence d'ions hydroxydes, sur une résine polymère insoluble en milieu basique aqueux et répondant à la formule générale suivante :

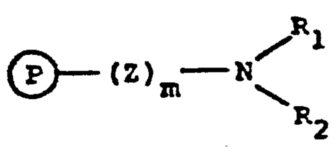

dans laquelle :

Ⓟ représente une matrice de résine polymère,

Z représente le groupe de formule

$$-NH-(CH_2)_n-\underset{\underset{O}{\|}}{C}-$$

avec $1 \leqslant n \leqslant 5$,

$m = 0$ ou 1,

$R_1$ représente un atome d'hydrogène,

$R_2$ représente un groupe cyclanone de 4 à 7 chaînons comportant éventuellement de 1 à 3 hétéroatomes d'azote ou un groupe de formule :

$$-\overset{R'}{\underset{R'}{C}}-(CH_2)_{n'}-\underset{\underset{O}{\|}}{C}-R$$

dans laquelle :

R' désigne un atome d'hydrogène ou un radical méthyle ou éthyle,

R désigne un radical méthyle ou éthyle, et

n′ = 0 à 3,
ou bien $R_1$ et $R_2$ forment ensemble avec l'atome d'azote voisin un groupe cyclanone de 5 à 7 chaînons comportant éventuellement 1 ou 2 hétéroatomes additionnels d'azote, et pouvant éventuellement être substitués par un ou plusieurs radicaux méthyle ou éthyle, et en ce que de manière en soi connue, les ions hydroxydes sont introduits dans le milieu réactionnel aqueux à raison de 0,1 à 0,3 mole par mole d'alpha-amino-nitrile de départ pour obtenir l'alpha-amino-amide correspondant, ou bien les ions hydroxydes sont introduits dans le milieu réactionnel aqueux de manière à atteindre sensiblement l'équimolarité par rapport à l'alpha-amino-nitrile de départ pour obtenir l'alpha-amino-acide correspondant.

Selon une autre caractéristique du procédé de la présente invention, le milieu réactionnel aqueux comporte une teneur minimale en eau correspondant à 1 mole d'eau par mole d'α-amino-nitrile de départ.

Selon une autre caractéristique additionnelle du procédé d'hydrolyse de la présente invention, on fait réagir un mélange d'aldéhyde, d'acide cyanhydrique et d'ammoniaque ou encore un mélange de cyanhydrine et d'ammoniaque, tous deux générateurs d'α-amino-nitrile, en présence d'ions hydroxydes sur une résine polymère qui comporte des chaînes latérales se terminant par un groupement carbonylé, et qui est insoluble en milieu basique aqueux.

Le procédé d'hydrolyse catalytique chimique selon l'invention s'applique en particulier à des α-amino-nitriles répondant à la formule générale suivante :

$$R''-\underset{\displaystyle NH_2}{\underset{|}{CH}}-C\equiv N$$

dans laquelle, le radical R″ représente un atome d'hydrogène ou une chaîne hydrocarbonée, linéaire ou ramifiée, contenant de 1 à 12 atomes de carbone et éventuellement un ou plusieurs hétéroatomes, tels que le soufre, ledit radical R″ étant éventuellement substitué une ou plusieurs fois, de préférence en fin de chaîne, par des groupements tels que hydroxy, amino, carboxyle, phényle, hydroxyphényle, carboxamide, indolyle, iminazyle et guanidyle, ou bien R″ forme avec l'atome d'azote en position α un groupe hétérocyclique saturé contenant au moins un hétéroatome, tel que l'azote, ledit groupe hétérocyclique pouvant lui-même être substitué, par exemple par un groupe hydroxy, ainsi qu'à leurs sels et à leurs précurseurs.

Le procédé de la présente invention s'applique en particulier à l'α-amino-propionitrile conduisant à l'alanine, ainsi qu'à l'α-aminométhylmercaptobutyronitrile, précurseur de la méthionine qui est un acide aminé d'un très grand intérêt économique, ou encore aux sels de ces nitriles tels que les chlorhydrates, ainsi qu'à leurs précurseurs.

D'autres caractéristiques et avantages de la présente invention apparaîtront à la lecture de la description détaillée faite ci-après, notamment en référence à quelques exemples de mise en œuvre particuliers.

Conformément au procédé de la présente invention, on constate qu'en introduisant un α-amino-nitrile ou l'un de ses sels, tels que par exemple le chlorhydrate, dans une solution aqueuse contenant une suspension d'une résine polymère carbonylée insoluble et des ions hydroxydes, on obtient très rapidement et de façon quantitative, même à la température ambiante, l'α-amino-amide correspondant à l'α-amino-nitrile de départ. La résine carbonylée insoluble peut alors être séparée du milieu réactionnel par simple filtration ou centrifugation, puis recyclée telle quelle sans aucun processus de régénération.

Au lieu d'être effectué de façon discontinue, avec recyclage du catalyseur, le procédé peut également être mis en œuvre de façon continue par contact d'une solution d'α-amino-nitrile ou de l'un de ses sels avec le catalyseur carbonylé insolubilisé et immobilisé. Selon une telle variante avantageuse du procédé de l'invention, l'α-amino-nitrile est par exemple introduit en tête d'un réacteur tubulaire, en même temps qu'une solution d'ions hydroxydes à raison de 0,1 à 1 mole d'hydroxyde par mole d'α-amino-nitrile de départ. La résine polymère carbonylée pourra par exemple être immobilisée dans le réacteur tubulaire et présenter une capacité de 0,1 à 10 milliéquivalents de fonction carbonyle par gramme de catalyseur. L'α-amino-amide peut ainsi être récupéré directement à la sortie du réacteur. Il est également possible d'introduire dans l'effluent des ions hydroxydes, afin d'atteindre l'équimolarité par rapport à l'α-amino-amide qui est ainsi hydrolysé quantitativement en l'α-amino-acide correspondant.

Les ions hydroxydes sont apportés dans le milieu réactionnel sous forme d'un hydroxyde d'un métal alcalin ou alcalino-terreux ou encore sous forme d'hydroxyde d'ammonium.

De façon plus particulière, les résines polymères porteuses de chaînes latérales à groupement carbonylé terminal répondant aux formules générales suivantes I, II et III, sans pour autant que ces dernières soient limitatives, conviennent parfaitement dans la pratique pour la mise en œuvre du procédé selon l'invention.

Les résines polymères porteuses de chaînes latérales à groupement carbonylé terminal de type linéaire répondent à la formule générale I :

$$\text{Ⓟ}-(Z)_m-NH-\overset{\displaystyle R'}{\underset{\displaystyle R'}{\overset{|}{\underset{|}{C}}}}-(CH_2)_{n'}-\underset{\displaystyle O}{\underset{\|}{C}}-R \qquad (I)$$

dans laquelle :

Ⓟ représente une matrice de résine polymère,

Z représente le groupe de formule

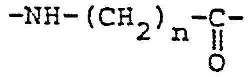

avec $1 \leqslant n \leqslant 5$,

R' représente un atome d'hydrogène ou un radical méthyle ou éthyle,

R représente un radical méthyle ou éthyle,

m = 0 ou 1, et

n' = 0 à 3.

Les résines polymères portant un groupement carbonylé se présentant sous la forme d'un groupement cyclanone, répondent à la formule générale II :

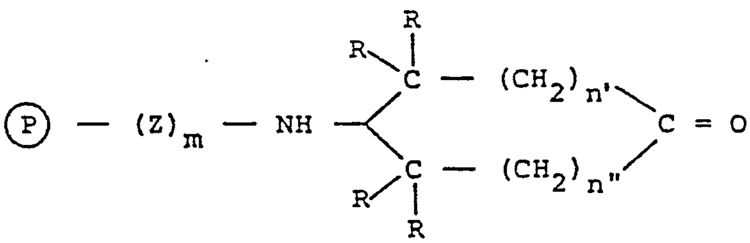

(II)

dans laquelle :

Ⓟ représente une matrice de résine polymère,

Z représente le groupe de formule

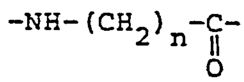

avec $1 \leqslant n \leqslant 5$,

R représente un atome d'hydrogène ou un radical méthyle ou éthyle,

m = 0 ou 1,

n' = 0 à 3,

n" = 0 à 3, et

$n' + n'' \leqslant 3$.

Les résines polymères porteuses d'un groupement carbonylé se présentant sous la forme d'un groupe hétérocyclanone répondent à la formule générale III :

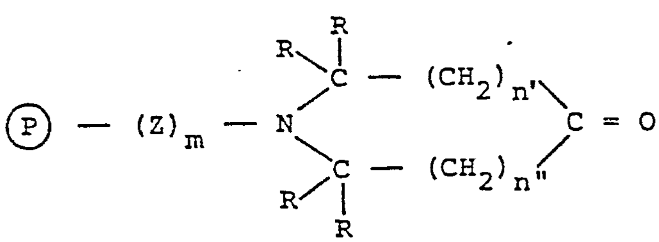

(III)

dans laquelle :

Ⓟ représente une matrice de résine polymère,

Z représente le groupe de formule

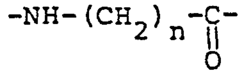

avec $1 \leqslant n \leqslant 5$,

R représente un atome d'hydrogène ou un radical méthyle ou éthyle,

m = 0 ou 1,

n' = 0 à 3,
n'' = 0 à 3, et
n' + n'' ≤ 3.

Dans les résines polymères de formule générale II, le groupe cyclanone de formule :

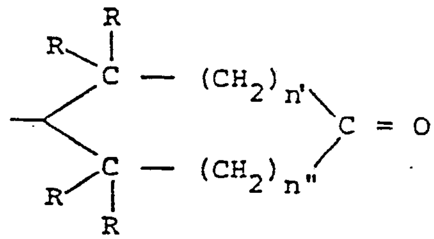

représente notamment les radicaux

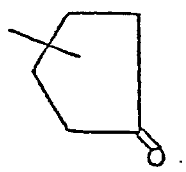 , 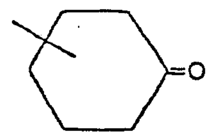 ou 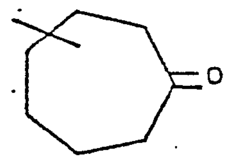

Dans les résines polymères de formule générale III, le groupe hétérocyclanone de formule :

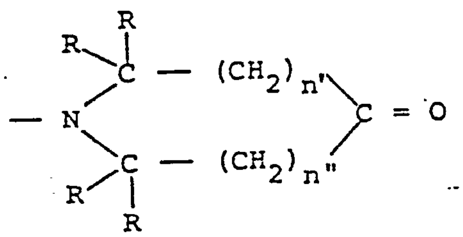

peut notamment être choisi parmi les radicaux suivants :

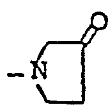 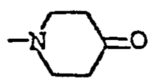 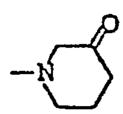 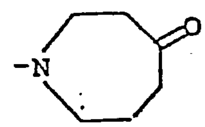 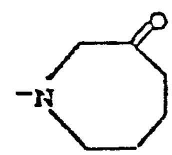

Conformément à la présente invention, la matrice de résine Ⓟ est avantageusement de type polystyrénique, polyacrylique ou encore cellulosique. Ces matrices sont avantageusement réticulées de manière à conduire à un polymère macroporeux ou à un gel se présentant par exemple sous la forme de grains ou de perles de résine rigides. Dans la suite du texte on adoptera le symbolisme suivant pour désigner les matrices de résine polymère Ⓟ :

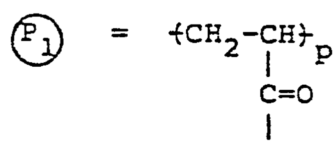

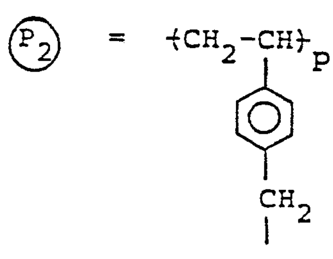

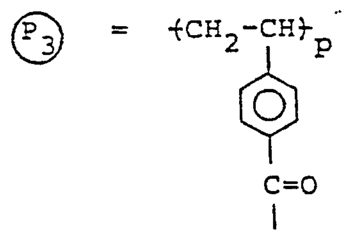

Ces différents types de matrice polymère P sont avantageusement réticulés par exemple avec du divinylbenzène ou avec une N,N' bis acryloyl polyméthylène diamine répondant à la formule générale (A)

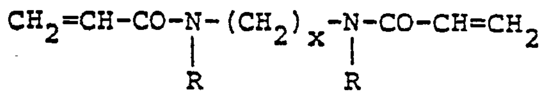
(A)

dans laquelle :

x = 1 à 6

R = H, $-CH_3$ ou $-CH_2-CH_3$

ou les deux radicaux R forment ensemble un groupe alcoylène de formule $(-CH_2-)_y$, et

y = 1 à 4.

On adoptera en particulier le symbolisme suivant :

($P_{1a}$) = ($P_1$) réticulé avec du divinylbenzène

($P_{1b}$) = ($P_1$) réticulé avec une N,N'bis acryloyl polyméthylène diamine de formule générale (A) définie ci-dessus

($P_2$) et ($P_3$) sont de préférence réticulés avec le divinylbenzène.

Les diverses résines polymères précitées peuvent être obtenues aussi bien par polymérisation de monomères que par fonctionnalisation d'un polymère.

La résine polymère carbonylée peut être préparée par polymérisation d'un monomère susceptible de générer par toute technique en soi connue une fonction carbonyle. A titre d'exemple on indiquera ci-après le schéma réactionnel de la préparation d'une résine polymère carbonylée selon l'invention de formule IIIc ou IIId, dans laquelle m = 0, n' = n" = 1 et R = H. Il s'agit donc d'une résine polymère dont la matrice est de type polyacrylique réticulé avec une N,N'bis acryloyl polyméthylène diamine de formule générale (A) indiquée précédemment.

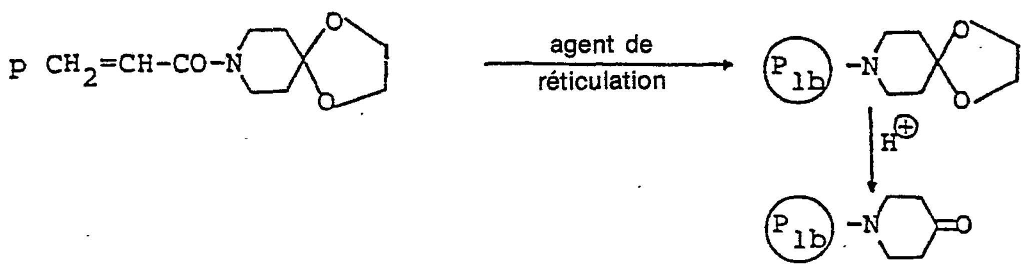


(IIIC)

Les résines polymères carbonylées selon l'invention peuvent également être préparées par greffage d'un composé carbonylé sur un polymère à matrice polyacrylique, polystyrénique ou encore cellulosique.

L'activation des polymères peut être obtenue par divers moyens en soi connus, par exemple dans le cas d'une résine polyacrylique par transformation des groupes carboxyle en groupes chlorure d'acide, ou encore par introduction de groupements chlorométhylés sur le noyau aromatique de polymère de type polystyrénique. On fait ensuite réagir ces polymères activés avec une molécule portant d'une part le groupement amine, et d'autre part une fonction susceptible de générer par tout processus en soi connu une fonction carbonyle. Le greffage s'effectue donc par formation d'une liaison covalente du type amide ou du type amine.

On notera que la fonction cétone de la molécule greffée peut être apparente, protégée par exemple sous la forme d'un cétal, ou encore latente par exemple sous la forme de groupements hydroxyles secondaires que l'on oxyde ultérieurement en groupe carbonyle.

Le groupement carbonylé, responsable de l'activité catalytique de la résine polymère, peut être plus ou moins éloigné du squelette de la matrice polymère, en intercalant une molécule d'acide aminé de formule $NH_2-(CH_2)_n-CO_2H$. Dans les formules générales précitées cet acide aminé apparaît sous la dénomination Z.

On rappellera ici brièvement que la capacité en groupements spécifiques d'une résine catalytique peut être par exemple exprimée en milliéquivalents/g (meq/g). Dans le cadre de la présente invention, lorsque l'on parle d'une résine ayant une capacité en sites carbonylés de x meq/g, cela signifie qu'un gramme de résine contient $x \cdot 10^{-3}$ équivalent molaire de groupement

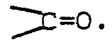

Dans la pratique, il s'est avéré que la capacité de la résine polymère en sites carbonylés à activité catalytique pouvait être avantageusement comprise entre 0,5 et 10 meq/g de polymère.

A titre d'illustration, on mentionnera ci-après quelques exemples particuliers de préparation de catalyseurs cétoniques par modification chimique de matrice de résine polymère.

A. Activation de la résine acrylique ($P_{1a}$) —OH

Un mélange de résine acrylique (10 g ; 0,10 eq.) réticulée avec 30 % de divinylbenzène, de chlorure de thionyle (20 $cm^3$ ; 0,27 mole), de diméthylformamide anhydre (8 $cm^3$), dans 100 $cm^3$ de chloroforme anhydre est chauffé à 60 °C pendant 6 heures sous agitation mécanique. Après filtration, la résine est lavée successivement par le chloroforme, l'éther anhydre, puis séchée sous pression réduite. Capacité en groupements chlorure d'acide : 5,0 meq/g. La résine utilisée peut par exemple être constituée par une résine polyacrylique commercialisée par les laboratoires BIO-RAD sous la dénomination Bio-Rex 70 (200-400 mesh). Elle se présente sous forme de sel de sodium et contient environ 70 % d'eau. De préférence préalablement à toute fonctionnalisation, on la débarrasse des impuretés résiduelles, de l'eau, et on la fait passer sous forme acide.

B. Préparation de la résine IIIa (Ⓟ = ($P_{1a}$) , m = 0 ; n' = n'' = 1, R = H)

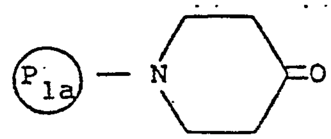 (IIIa)

La résine chlorure d'acide précédente (3,0 g ; 0,015 eq.) est ajoutée à une solution d'aza-8-dioxa-1,4-spiro [4-5] décane (4,3 g ; 0,030 mole) et de triéthylamine (1,6 g ; 0,016 mole) dans 65 $cm^3$ de diméthylformamide. Le mélange est agité pendant 7 heures à température ambiante. Après filtration, la résine est lavée à l'eau distillée puis mise en suspension et agitée pendant 3 heures dans un mélange soude diluée-diméthylformamide. On filtre, lave la résine à l'eau distillée, et la met en suspension dans de l'acide chlorhydrique 4 N. On agite pendant 6 heures à température ambiante. Après filtration, la résine est lavée successivement par l'eau distillée, l'éthanol et l'éther, puis séchée sous pression réduite. Capacité en groupements carbonyles : 2,5 meq/g.

C. Préparation de la résine IIIb (Ⓟ = ($P_{1a}$) , m = 1, n = 2, n' = n'' = 1, R = H)

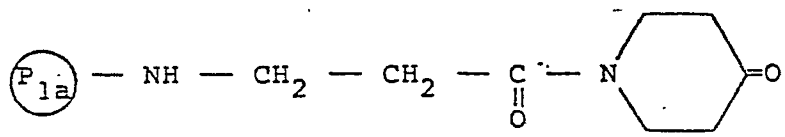 (IIIb)

La résine chlorure d'acide précédente (3,0 g ; 0,015 eq.) est ajoutée à une solution de β-alanine (5,35 g ; 0,06 mole) dans 36 $cm^3$ d'un mélange DMF-eau-N-éthylmorpholine 1/3 / 1/3 / 1/3. Après 17 heures d'agitation à température ambiante, on filtre. La résine est lavée successivement par l'eau distillée, l'acide chlorhydrique dilué, l'eau, l'éthanol et l'éther, puis séchée sous pression réduite.

3,6 g de cette résine sont mis à réagir dans une solution de chlorure de thionyle (8 $cm^3$ ; 0,11 mole) et de diméthylformamide anhydre (3 $cm^3$) dans 40 $cm^3$ de chloroforme anhydre. Le mélange est chauffé à 60 °C pendant 6 heures sous agitation mécanique. Après filtration, la résine est lavée successivement par le chloroforme, l'éther anhydre, puis séchée sous pression réduite. Capacité totale en groupements chlorure d'acide : 4,3 meq/g.

Cette résine (3,2 g ; 0,014 eq.) est ajoutée à une solution d'aza-8-dioxa-1,4-spiro [4-5] décane (4,5 g ; 0,032 mole) et de triéthylamine (1,8 g ; 0,018 mole) dans 70 $cm^3$ de diméthylformamide. Le mélange est agité pendant 9 heures à température ambiante puis filtré. La résine est lavée à l'eau distillée, mise en suspension et agitée pendant 3 heures dans un mélange soude diluée-diméthylformamide.

On filtre, lave la résine à l'eau distillée, et la met en suspension dans de l'acide chlorhydrique 4 N. On agite pendant 6 heures à température ambiante. Après filtration, la résine est lavée successivement par

l'eau distillée, l'éthanol et l'éther, puis séchée sous pression réduite. Capacité en groupements carbonyles : 2.0 meq/g.

D. Préparation de la résine IIa (Ⓟ = ($P_{1a}$), m = 0, n = n' = 1, R = H)

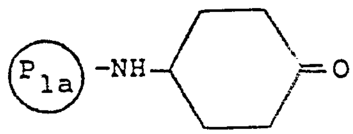

(IIa)

A une solution d'amino-4 cyclohexanol (3,0 g ; 0,026 mole) et de triéthylamine (2,9 g ; 0,029 mole) dans 60 $cm^3$ de diméthylformamide, on ajoute une résine chlorure d'acide de capacité 3,8 meq/g (4,0 g ; 0,015 eq.). Le mélange est agité pendant 7 heures à température ambiante, puis filtré. La résine est lavée successivement par le DMF, l'eau distillée, l'éthanol et l'éther. Après séchage sous pression réduite, 1,0 g de résine ainsi obtenue est oxydé de la façon suivante : on l'ajoute à une solution d'anhydride chromique (0,9 g : $9 \cdot 10^{-3}$ mole) dans 40 $cm^3$ de diméthylformamide, contenant quelques gouttes d'acide sulfurique concentré. Le mélange est agité pendant 19 heures à température ambiante, puis filtré. La résine est lavée successivement par l'eau, l'éthanol, un mélange soude diluée-éthanol, puis l'eau. Elle est ensuite acidifiée par des lavages à l'acide chlorhydrique dilué suivis de lavages à l'eau distillée, à l'éthanol et à l'éther. La résine séchée sous pression réduite a une capacité en groupements carbonyles de 1,2 meq/g.

E. Préparation de la résine Ia (Ⓟ = ($P_{1a}$), m = 0, n' = 0, R = Me, R' = H)

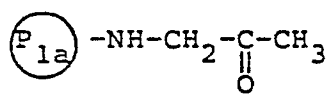

(Ia)

A une solution d'amino-1 propanol-2 (2,3 g ; 0,30 mole) et de triéthylamine (3,3 g ; 0,030 mole) dans 70 $cm^3$ de diméthylformamide, on ajoute une résine chlorure d'acide de capacité 3,8 meq/g (5,0 g ; 0,019 eq). Le mélange est agité pendant 7 heures à température ambiante, puis filtré. La résine est lavée successivement par le DMF, l'eau distillée, l'éthanol et l'éther. Après séchage sous pression réduite, on procède à l'oxydation : 5,9 g de résine alcool précédente sont ajoutés à une solution d'anhydrique chromique (10 g, 0,1 mole) dans 120 $cm^3$ de diméthylformamide, contenant quelques gouttes d'acide sulfurique concentré. Le mélange est agité 20 heures à température ambiante, puis filtré. La résine est lavée successivement par l'eau, l'éthanol, un mélange soude diluée-éthanol, puis l'eau distillée. Elle est ensuite acidifiée par des lavages à l'acide chlorhydrique dilué suivis de lavages à l'eau distillée, à l'éthanol et à l'éther. La résine séchée sous pression réduite a une capacité en groupements carbonyles de 0,85 meq/g.

On indiquera en outre ci-après un exemple de préparation des catalyseurs cétoniques par polymérisation.

Préparation du N-acryloyl aza-8 dioxa-1,4 spiro [4-5] décane

Une solution d'aza-8 dioxa-1,4 spiro [4-5] décane (28,6 g ; 0,20 mole) dans de l'éther anhydre (100 $cm^3$) est agitée et refroidie à 0 °C. On additionne goutte à goutte le chlorure d'acryloyle (9,06 g ; 0,10 mole) en solution dans l'éther anhydre (33 $cm^3$). Deux heures après le début de l'addition, on élimine le précipité blanc par filtration. Par évaporation de l'éther sous pression réduite on obtient le produit brut (rendement = 97 %) qui est ensuite distillé ($Eb_{0,06}$ = 110-114 °C) (rendement = 85 %). F = 43-44 °C (pentane-éther).

Préparation du copolymère de N-acryloyl aza-8 dioxa-1,4 spiro [4-5] décane et de N,N'-méthylenebisacrylamide (rapport molaire 6/1)

Obtention du copolymère IIIc (Ⓟ = ($P_{1b}$), m = 0, n' = n" = 1, R = H)

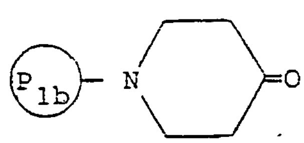

(IIIc)

On fait passer un courant d'azote pendant 15 minutes dans une solution de N-acryloyl aza-8 dioxa-1,4 spiro [4-5] décane (5,46 g ; 0,028 mole) et de N,N'-méthylènebisacrylamide (0,71 g ; 0,004 6 mole) dans de

l'eau distillée (18 $cm^3$). Après addition de quelques milligrammes de persulfate de potassium et de riboflavine, la solution est soumise pendant 10 minutes aux radiations d'une lampe de 500 watts.

Le polymère obtenu est lavé à l'eau, à l'éthanol, à l'éther, puis séché sous pression réduite.

On agite ensuite pendant 24 heures une suspension du polymère obtenu dans de l'acide chlorhydrique 1 N. Le polymère est recueilli par filtration, lavé à l'eau, à l'éthanol, à l'éther puis séché sous pression réduite, sa capacité en groupements carbonyle est de 4,0 meq/g.

Préparation du copolymère de N-acryloyl aza-8 dioxa-1,4 spiro [4-5] décane et de N,N' bis acryloyl-pipérazine (rapport molaire 4,5/1)

Obtention du copolymère IIId (Ⓟ = $(P_{1b})$ , m = 0, n' = n" = 1, R = H)

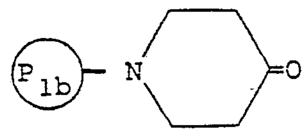

(IIId)

On fait passer un courant d'azote pendant 30 minutes dans une solution de N-acryloyl aza-8 dioxa-1,4 spiro [4-5] décane (5 g ; 0,025 mole) et de N,N'bis acryloyl pipérazine (1,1 g ; 0,005 6 mole) dans de l'eau distillée (14 $cm^3$). On refroidit à 0 °C, on ajoute 56 mg de persulfate d'ammonium solubilisés dans de l'eau distillée (2 $cm^3$), et 50 microlitres de N,N' tétraméthyl éthylène diamine (TEMED). On maintient à 0° pendant 1 heure 30. Le polymère obtenu est broyé au mortier, lavé à l'eau, à l'éthanol, à l'éther puis séché sous pression réduite. On agite pendant 24 heures une suspension du copolymère dans de l'acide chlorhydrique 1 N. Le polymère est recueilli par filtration, lavé à l'eau, à l'éthanol, puis séché sous pression réduite. Sa capacité en groupements carbonyle est de 4,2 meq/g.

Préparation du copolymère de N-acryloyl aza-8 dioxa-1,4 spiro [4-5] décane et de N,N'bis acryloyl pipérazine en perles (rapport molaire 5/1)

Obtention du copolymère IIIe (Ⓟ = $(P_{1b})$ , m = 0, n' = n" = 1, R = H)

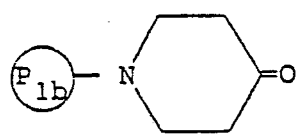

(IIIe)

100 ml d'huile de paraffine contenant 2 gouttes de sorbitan trioléate sont soumis à un courant d'azote pendant 2 heures. Une solution de N-acryloyl aza-8 dioxa-1,4 spiro [4-5] décane (1,8 g ; 0,009 15 mole) et de N,N'bis acryloyl pipérazine (0,35 g ; 0,001 83 mole) dans l'eau distillée (7,5 $cm^3$) est également soumise à un courant d'azote pendant 1 heure. On ajoute ensuite à la solution aqueuse 20 microlitres de N,N' tétraméthyl éthylène diamine (TEMED) et 25 mg de persulfate d'ammonium solubilisés dans de l'eau distillée (1 $cm^3$). On verse ensuite la solution aqueuse dans l'huile de paraffine puis agite sous atmosphère d'azote pendant 1 heure. On filtre ensuite le polymère sous forme de perles, on lave au pentane puis à l'eau distillée.

Le polymère obtenu, est agité en suspension 24 heures dans de l'acide chlorhydrique 1 N. Il est ensuite recueilli par filtration, puis lavé à l'eau. Sa capacité en groupements carbonyle est de 4,1 meq/g.

Les copolymères décrits ci-dessus doivent être obligatoirement séchés avant de procéder aux caractérisations analytiques. Dans le cas de préparations de routine, on peut se dispenser de ces opérations, les lavages à l'eau suivis d'essorage suffisent.

Efficacité des catalyseurs

L'efficacité des résines polymères cétoniques selon l'invention a été testée sur divers α-amino-nitriles dans un réacteur tubulaire après avoir été conditionnés par agitation dans une solution de soude diluée. A titre d'exemple, on indiquera dans le tableau A ci-après quelques résultats relatifs à l'hydratation de l'α-amino-propionitrile.

Dans le tableau A ci-après les conditions opératoires sont les suivantes :

température du réacteur = 25 °C

Concentration en α-aminopropionitrile à l'entrée du réacteur tubulaire : 0,05 mole/l.

Concentration en hydroxyde : 0,02 mole/l.

(Voir Tableau A p. 10)

Tableau A

Etude comparative de l'efficacité de différents polymères cétoniques (réacteur contenant 1,3g de catalyseur, et 4,5 $cm^3$ de solution)

| Polymère cétonique | Capacité du polymère en groupements carbonyles exprimé en milli-équivalent par gramme de catalyseur | Temps de contact dans le réacteur en minute | Taux de conversion en pourcentage d'α-aminopropionamide en sortie de réacteur par rapport à l'α-aminopropionitrile injecté |
|---|---|---|---|
| Ia<br>(Ⓟ = ($P_{1a}$), m=0, n"=0, R=Me, R'=H) | 0,85 | 66<br>33<br>14,6 | 26<br>14,5<br>6,8 |
| IIa<br>(Ⓟ = ($P_{1a}$), m=0, n=n'=1, R=H) | 1,2 | 14,6<br>8,8 | 12<br>8,5 |
| IIIa<br>(Ⓟ = ($P_{1a}$), m=0, n'=n"=1, R=H) | 1,5 | 33<br>14,6<br>8,8<br>6,2 | 77<br>34<br>25<br>17 |
| IIIb<br>(Ⓟ = ($P_{1a}$), m=1, n=2, n'=n"=1, R=H) | 2 | 33<br>14,6<br>8,8<br>6,2 | 74<br>33<br>20<br>14 |
| IIIc ou IIId<br>(Ⓟ = ($P_{1b}$), m=0, n'=n"=1, R=H) | 3 | 33<br>14,6<br>8,8 | 99,8<br>93,4<br>80,6 |

Il convient ici de rappeler que dans le procédé relatif à l'hydrolyse catalytique chimique d'α-amino-nitriles en milieu homogène, les cétones cycliques ne sont pas utilisables, car elles se dégradent rapidement en milieu basique, notamment par réaction d'aldolisation-crotonisation. Ainsi, on a pu observer la formation de cyclohexylidène-2-cyclohexanone, lors de l'hydratation des α-amino-nitriles catalysés par la cyclohexanone :

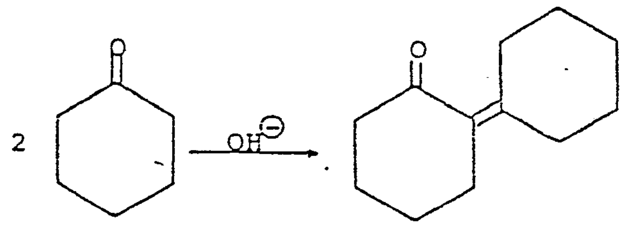


En revanche, conformément au procédé de la présente invention, de telles réactions secondaires entre les molécules cétoniques sont beaucoup plus limitées lorsqu'elles sont immobilisées sur une matrice polymère. On notera à ce sujet que, dans la pratique, les cétones cycliques insolubilisées sont apparues plus efficaces que les cétones aliphatiques linéaires.

Outre la nature exacte du motif cétonique à activité catalytique, les caractéristiques physico-chimiques de la matrice de résine polymère (agent de réticulation, degré de réticulation, caractère plus ou moins hydrophile) interviennent de façon notable, notamment en raison de l'importance des processus de diffusion en catalyse hétérogène.

La nature du polymère catalyseur étant fixée, les paramètres importants définissant le taux de conversion de l'α-amino-nitrile en α-amino-amide en sortie de réacteur, ont été mis en évidence.

A titre indicatif on mentionnera ci-après quelques résultats relatifs à l'hydratation de l'α-amino-propionitrile catalysé par le polymère carbonylé de formule IIIa dans laquelle Ⓟ = $(P_{1a})$ m = 0, n = n' = 1, R = H, c'est-à-dire le polymère de formule :

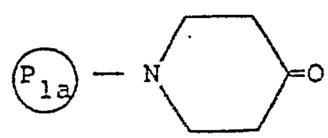

Ces différents paramètres ont été regroupés dans le tableau B ci-après :

(Voir tableau B p. 12)

L'examen de ce tableau révèle que le taux de conversion de l'α-amino-nitrile en α-amino-amide croît :

— avec le nombre de sites carbonylés catalyseurs par unité de masse de polymère ;
— avec la concentration en ions hydroxydes dans le réacteur,
— avec la température du réacteur, et
— avec le temps de séjour de l'α-amino-nitrile au contact du catalyseur, pour un type de réacteur donné et pour une concentration en α-amino-nitrile donné, le taux de conversion étant une fonction exponentielle du temps de séjour.

Au cours de ces expérimentations il est notamment apparu que le polymère cétonique insolubilisé joue son rôle de catalyseur quel que soit le nombre d'équivalents cétoniques introduits. De préférence, conformément au procédé de l'invention, la solution d'α-amino-nitrile est mise au contact du polymère carbonylé insolubilisé à raison de 0,1 à 50 équivalents de composés carbonylés par mole d'α-amino-nitrile de départ.

Dans la pratique, il est également apparu que la résine polymère pouvait être introduite dans le milieu réactionnel à raison d'environ 10 g à environ 2 000 g de résine par mole d'α-amino-nitrile de départ, par exemple dans le cas d'une mise en œuvre du procédé selon l'invention de façon discontinue, avec recyclage du catalyseur carbonylé.

Il a par ailleurs été constaté que la résine polymère carbonylée pouvait avantageusement porter, en plus des chaînes latérales à groupement carbonylé, des groupes fonctionnels hydrophiles, tels que par exemple des groupements carboxyliques, amines primaire, secondaire, tertiaire ou ammoniums quaternaires.

On précisera enfin que la température du milieu réactionnel sera sensiblement comprise entre environ 5 °C et environ 80 °C.

Tableau B : Hydratation de l'α-aminopropionitrile en α-aminopropioamide dans un réacteur tubulaire rempli de catalyseur IIIa (1,3 g de catalyseur et 4,5 cm³ de solution) (Ⓟ = $P_{1a}$ m = 0, n' = n" = 1, R = H).
Mise en évidence des paramètres importants

| Capacité du polymère en groupements carbonyles exprimé en milliéquivalent par gramme de catalyseur | Concentration en hydroxyde en mole/litre | Concentration en α-aminopropionitrile à l'entrée du réacteur en mole/litre | Temps de contact dans le réacteur en min. | Température du réacteur (°C) | Taux de conversion en % d'α-aminopropionamide en sortie de réacteur par rapport à l'α-aminopropionitrile injecté |
|---|---|---|---|---|---|
| 0 | 0,05 | 0,05 | 18 | 25 | 5 |
| 1,4 | " | " | " | " | 73,5 |
| 1,8 | " | " | " | " | 84,5 |
| 1,5 | 0,02 | 0,05 | 14,6 | 25 | 33,5 |
| " | 0,05 | " | " | " | 63,5 |
| " | 0,10 | " | " | " | 79 |
| " | 0,15 | " | " | " | 87 |
| 1,5 | 0,05 | 0,025 | 14,6 | 25 | 68,5 |
| " | " | 0,050 | " | " | 63,5 |
| " | " | 0,075 | " | " | 57,5 |
| " | " | 0,10 | " | " | 55 |
| 1,5 | 0,05 | 0,05 | 33 | 25 | 85 |
| " | " | " | 14,6 | " | 63,5 |
| " | " | " | 8,8 | " | 52 |
| " | " | " | 5,1 | " | 35 |
| 1,8 | 0,07 | 0,10 | 8,5 | 10 | 34 |
| 1,8 | " | " | " | 30 | 76 |
| 1,8 | " | " | " | 40 | 98 |

Le procédé objet de la présente invention peut être mis en œuvre par exemple de la manière suivante. La résine polymère cétonique, dont les groupements carboxyles résiduels sont sous forme acide, doit être de préférence au préalable conditionnée. On agite par exemple pendant plusieurs heures dans de la soude 0,1 N avant de procéder au remplissage de la colonne de catalyseur.

Deux seringues de diamètre identique, actionnées par le même moteur, alimentent avec le même débit la colonne de résine par sa partie inférieure. L'une des seringues contient par exemple le chlorhydrate de l'α-amino-nitrile en solution dans de l'acide chlorhydrique dilué, l'autre une solution de soude. A l'entrée de la colonne, par exemple en partie basse, la solution de soude libère l'α-amino-nitrile de son chlorhydrate et amène le milieu réactionnel au pH requis pour l'hydratation catalytique.

Pour éviter la réaction autocatalytique qui modifierait la composition des effluents avant l'analyse, on additionne en sortie de colonne une solution d'acide chlorhydrique diluée.

Selon une variante du procédé, l'α-amino-nitrile peut être préparé par l'action d'une cyanhydrine sur de l'ammoniaque. L'α-amino-nitrile de départ peut également être préparé par l'action d'un aldéhyde et d'acide cyanhydrique ou de cyanure tel qu'un cyanure alcalin, sur de l'ammoniaque et un sel d'ammonium. Dans la pratique, il peut être avantageux dans pareil cas de stabiliser la solution d'α-amino-nitrile par un excès d'environ 5 à 10 % de cyanure par rapport au cyanure contenu dans la solution équimolaire aldéhyde-cyanure ou par rapport à la solution de cyanhydrine.

Selon une variante du procédé de l'invention, on laisse s'installer l'équilibre de formation de l'α-amino-nitrile avant d'introduire le système catalytique constitué par la résine carbonylée en présence des ions hydroxydes.

On indiquera ci-après quelques exemples d'illustration du procédé objet de la présente invention.

## Exemple 1

Une solution de chlorhydrate d'α-aminopropionitrile 0,10 M dans HCl 0,01 N est injectée en même temps qu'un volume identique de soude 0,21 N en tête d'un réacteur contenant 1,4 g du polymère cétonique IIIa immobilisé, à $1{,}8 \cdot 10^{-3}$ équivalents de groupement cétonique par gramme de polymère. En sortie du réacteur l'α-aminopropionamide est obtenu avec un taux de conversion de 95 %. La productivité du réacteur fonctionnant à température ambiante est de $1{,}2 \cdot 10^{-3}$ mole/min. ramenée à 1 litre de réacteur et à 1 gramme de résine.

## Exemple 2

Une solution de chlorhydrate d'α-aminopropionitrile 0,10 M dans HCl 0,01 N est injectée en même temps qu'un volume identique de soude 0,21 N en tête d'un réacteur contenant 1,5 g de polymère cétonique IIa immobilisé à $1{,}2 \cdot 10^{-3}$ équivalents de groupement cétonique par gramme de polymère. En sortie de réacteur l'α-aminopropionamide est obtenu avec un taux de conversion de 90 %. La productivité de réacteur fonctionnant à 30 °C est $0{,}55 \cdot 10^{-3}$ mole/min. ramenée à 1 litre de réacteur et à 1 g de résine.

## Exemple 3

A 20 ml d'une solution 0,2 molaire d'α-amino-nitrile on ajoute 4 ml de soude 1 N, 1 g du polymère cétonique insoluble IIIa à 1,8 milliéquivalent par gramme puis on agite le mélange pendant 1 heure à température ambiante. Après neutralisation à l'acide chlorhydrique et centrifugation du mélange hétérogène, l'analyse en RMN du surnageant montre que le taux de transformation de l'α-aminopropionitrile en α-amino-amide est de 91 %.

## Exemple 4

Une solution de chlorhydrate d'α-aminométhylmercaptobutyronitrile 0,10 M dans HCl 0,01 N est injectée en même temps qu'un volume identique de soude 0,21 N en tête d'un réacteur contenant 1,4 g du polymère cétonique IIIa immobilisé à $1{,}8 \cdot 10^{-3}$ équivalents de groupement cétonique par gramme de polymère. En sortie de réacteur l'α-aminométhylmercaptobutyramide est obtenu avec un taux de conversion de 95 %. La productivité du réacteur fonctionnant à température ambiante est de $0{,}57 \cdot 10^{-3}$ mole/min. ramenée à 1 litre de réacteur et à 1 gramme de résine.

## Exemple 5

0,866 g d'acétaldéhyde ($2 \cdot 10^{-2}$ moles) sont ajoutés à 100 ml d'une solution de $NH_4OH$ 5 N contenant 1,284 g de $ClNH_4$ (0,024 0 mole) et 1,432 g de KCN (0,022 0 mole). Le mélange est maintenu dans un flacon bouché à 40 °C pendant 1/2 heure. Ce mélange en même temps qu'un volume identique d'une solution de soude 0,1 N, est injecté en tête d'un réacteur contenant 1,4 g du polymère cétonique IIIa, immobilisé à $1{,}8 \cdot 10^{-3}$ équivalents de groupements cétoniques par gramme de polymère. En sortie de réacteur l'α-amino-amide est obtenu avec un taux de conversion de 95 %. Dans ces conditions, la

productivité du réacteur fonctionnant à température ambiante est de $1,0 \cdot 10^{-3}$ mole/min. ramenée à 1 litre de réacteur et à 1 gramme de résine.

Au mélange réactionnel issu du réacteur on ajoute 10 ml de soude normale de façon à obtenir l'équimolarité des ions hydroxydes et de l'α-amino-amide. Le mélange est alors chauffé à 80 °C à l'air libre pendant 1/2 heure. Le dosage de l'alanine par analyseur d'amino acides indique un rendement de 90 %.

Exemple 6

A 100 ml d'une solution 0,22 molaire en $ClNH_4$ et 0,22 molaire en KCN dans $NH_4OH$ 10 N on ajoute 2,032 g (environ $2 \cdot 10^{-2}$ moles) de méthylmercaptopropionaldéhyde. Le mélange est chauffé à 40 °C sous agitation magnétique dans un erlen bouché pendant 1 heure et demie. Ce mélange en même temps qu'un volume identique d'une solution de soude 0,1 N est injecté en tête d'un réacteur contenant 1,4 g de polymère cétonique IIIa immobilisé à $1,8 \cdot 10^{-3}$ équivalents de groupement cétonique par gramme de polymère. En sortie de réacteur l'α-amino-amide est obtenu avec un taux de conversion de 95 % mesuré en chromatographie liquide haute pression. Dans ces conditions, la productivité du réacteur fonctionnant à 25 °C est de $0,52 \cdot 10^{-3}$ mole/min. ramenée à 1 litre de réacteur et à 1 gramme de résine.

Au mélange réactionnel issu du réacteur on ajoute 10 ml de soude 1 N de façon à obtenir l'équimolarité des ions hydroxydes et de l'α-aminoamide, le mélange est alors chauffé à 80 °C à l'air libre pendant 1 heure. Le dosage de la méthionine par la méthode NNR indique un rendement de 95 %.

Exemple 7

Une solution de chlorhydrate d'α-aminopropionitrile 0,05 M dans HCl 0,01 N est injectée en même temps qu'un volume identique de soude 0,11 N en tête d'un réacteur contenant 1,3 g du polymère cétonique IIIb immobilisé à $2,0 \cdot 10^{-3}$ équivalents de groupement cétonique par gramme de polymère. En sortie du réacteur, l'α-aminopropionamide est obtenu avec un taux de conversion de 85 %. La productivité du réacteur fonctionnant à température ambiante est de $0,49 \cdot 10^{-3}$ mole/min. ramenée à 1 litre de réacteur et à 1 gramme de résine.

Exemple 8

Une solution de chlorhydrate d'α-aminopropionitrile 0,10 M dans HCl 0,01 N est injectée en même temps qu'un volume identique de soude 0,15 N en tête d'un réacteur contenant 1,3 g du polymère cétonique IIIc immobilisé à $3,0 \cdot 10^{-3}$ équivalents de groupement cétonique par gramme de polymère. En sortie du réacteur, l'α-aminopropionamide est obtenu avec un taux de conversion de 99,1 %. La productivité du réacteur fonctionnant à température ambiante est de $1,53 \cdot 10^{-3}$ mole/min. ramenée à 1 litre de réacteur et à 1 gramme de résine.

Exemple 9

Une solution de chlorhydrate d'α-aminopropionitrile 0,10 M dans HCl 0,01 N est injectée en même temps qu'un volume identique de soude 0,21 N en tête d'un réacteur contenant 3,3 g du polymère cétonique Ia immobilisé à $0,85 \cdot 10^{-3}$ équivalent de groupement cétonique par gramme de polymère. En sortie du réacteur, l'α-aminopropionamide est obtenu avec un taux de conversion de 35 %. La productivité de réacteur fonctionnant à température ambiante est de $8 \cdot 10^{-5}$ mole/min. ramenée à 1 litre de réacteur et à 1 gramme de résine.

Exemple 10

A une suspension de 1,50 gramme de polymère cétonique IIId, à $2,6 \cdot 10^{-3}$ équivalents de groupements cétoniques par gramme de polymère dans 15 $cm^3$ d'une solution de soude 0,20 N sont ajoutés 80 mg de chlorhydrate d'α-aminopropionitrile. Le mélange est agité 10 minutes à 20 °C. Après neutralisation à l'acide chlorhydrique et centrifugation, l'analyse en RMN du surnageant révèle un taux de transformation de 97 % en α-aminopropionamide.

Bien entendu, la présente invention ne saurait être limitée aux modes de mise en œuvre particuliers du procédé décrit, mais il est parfaitement possible, sans pour autant sortir du cadre de la présente invention, d'en imaginer un certain nombre de variantes de détail. C'est ainsi qu'en particulier il sera possible d'apporter un certain nombre de modifications relatives à la structure et aux propriétés physico-chimiques de la résine polymère carbonylée.

**Revendications**

1. Procédé d'hydrolyse catalytique chimique d'un alpha-amino-nitrile ou de l'un de ses sels pour

obtenir l'amide ou l'acide correspondant, caractérisé en ce que l'on fait réagir en milieu aqueux ledit alpha-amino-nitrile ou l'un de ses sels, en phase hétérogène et en présence d'ions hydroxydes, sur une résine polymère insoluble en milieu basique aqueux et répondant à la formule générale suivante :

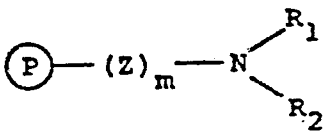

dans laquelle :

Ⓟ représente une matrice de résine polymère,

Z représente le groupe de formule

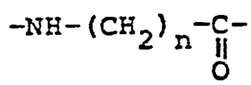

avec $1 \leqslant n \leqslant 5$,

$m = 0$ ou 1,

$R_1$ représente un atome d'hydrogène,

$R_2$ représente un groupe cyclanone de 4 à 7 chaînons comportant éventuellement de 1 à 3 hétéroatomes d'azote ou un groupe de formule :

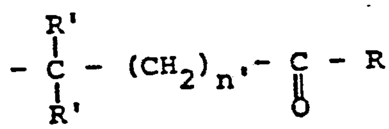

dans laquelle :

R' désigne un atome d'hydrogène ou un radical méthyle ou éthyle,

R désigne un radical méthyle ou éthyle, et

$n' = 0$ à 3,

ou bien $R_1$ et $R_2$ forment ensemble avec l'atome d'azote voisin un groupe cyclanone de 5 à 7 chaînons comportant éventuellement 1 ou 2 hétéroatomes additionnels d'azote, et pouvant éventuellement être substitués par un ou plusieurs radicaux méthyle ou éthyle, et en ce que de manière en soi connue, les ions hydroxydes sont introduits dans le milieu réactionnel aqueux à raison de 0,1 à 0,3 mole par mole d'alpha-amino-nitrile de départ pour obtenir l'alpha-amino-amide correspondant, ou bien les ions hydroxydes sont introduits dans le milieu réactionnel aqueux de manière à atteindre sensiblement l'équimolarité par rapport à l'alpha-amino-nitrile de départ pour obtenir l'alpha-amino-acide correspondant.

2. Procédé selon la revendication 1, caractérisé en ce que ladite résine polymère répond à la formule générale I

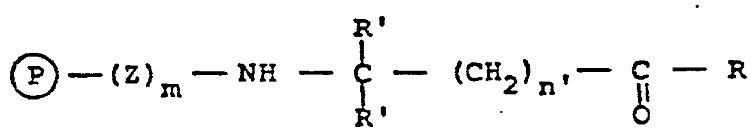

(I)

dans laquelle :

Ⓟ représente une matrice de résine polymère,

Z représente le groupe de formule

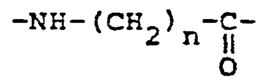

avec $1 \leqslant n \leqslant 5$,

R' représente un atome d'hydrogène ou un radical méthyle ou éthyle,

R représente un radical méthyle ou éthyle, et

$m = 0$ ou 1,

$n' = 0$ à 3.

3. Procédé selon la revendication 1, caractérisé en ce que ladite résine polymère répond à la formule générale II

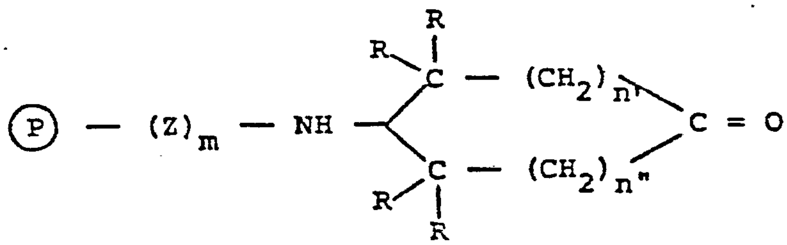

(II)

dans laquelle :

Ⓟ représente une matrice de résine polymère,

Z représente le groupe de formule

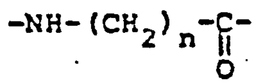

avec $1 \leqslant n \leqslant 5$,

R représente un atome d'hydrogène ou un radical méthyle ou éthyle,

m = 0 ou 1,

n' = 0 à 3,

n" = 0 à 3,

n' + n" ≤ 3.

4. Procédé selon la revendication 1, caractérisé en ce que ladite résine polymère répond à la formule générale III

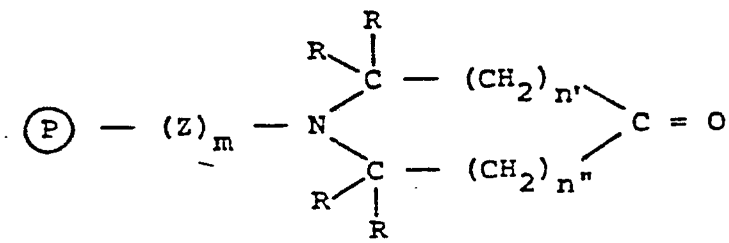

(III)

dans laquelle :

Ⓟ représente une matrice de résine polymère,

Z représente le groupe de formule

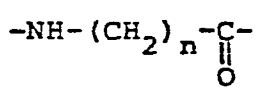

avec $1 \leqslant n \leqslant 5$,

R représente un atome d'hydrogène ou un radical méthyle ou éthyle,

m = 0 ou 1,

n' = 0 à 3,

n" = 0 à 3,

n' + n" ≤ 3.

5. Procédé selon la revendication 3, caractérisé en ce que ladite résine polymère répond à la formule générale II dans laquelle le groupe cyclanone de formule

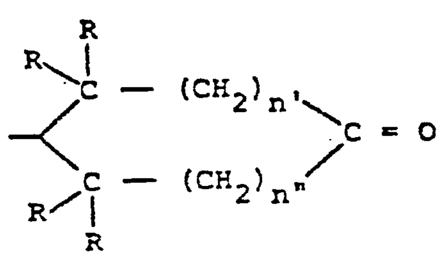

représente les radicaux

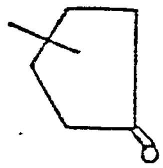 , 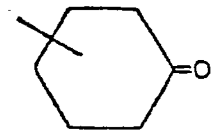 ou 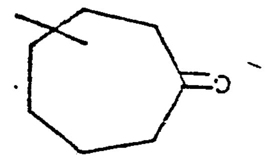

6. Procédé selon la revendication 4, caractérisé en ce que ladite résine polymère répond à la formule générale III dans laquelle le groupe hétérocyclanone de formule

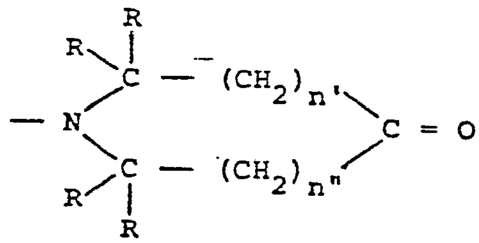

est choisi parmi les radicaux

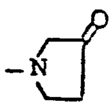 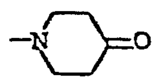 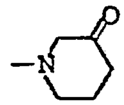 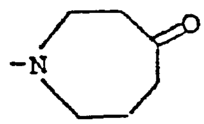 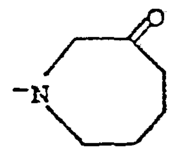

7. Procédé selon l'une des revendications 1 à 6, caractérisé en ce que la matrice de résine polymère Ⓟ est du type polystyrénique, polyacrylique ou cellulosique.

8. Procédé selon la revendication 7, caractérisé en ce que la matrice de résine polymère Ⓟ est du type polystyrénique ou polyacrylique réticulé.

9. Procédé selon la revendication 8, caractérisé en ce que la matrice de résine polymère Ⓟ est une résine polyacrylique de formule

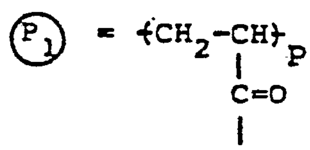

réticulée, en particulier avec du divinylbenzène ou avec une N,N'bis acryloyl polyméthylène diamine répondant à la formule générale (A) :

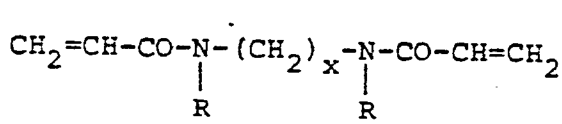 (A)

dans laquelle :

x = 1 à 6

R = H, $-CH_3$ ou $-CH_2-CH_3$

ou les deux radicaux R forment ensemble un groupe alcoylène de formule $(-CH_2-)_y$, et

y = 1 à 4.

10. Procédé selon la revendication 8, caractérisé en ce que la matrice de résine polymère Ⓟ est une résine de polystyrène de formule

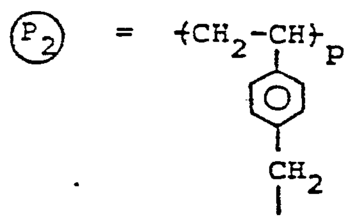

réticulée, en particulier avec du divinylbenzène.

11. Procédé selon la revendication 8, caractérise en ce que la matrice de résine polymère Ⓟ est une résine de formule

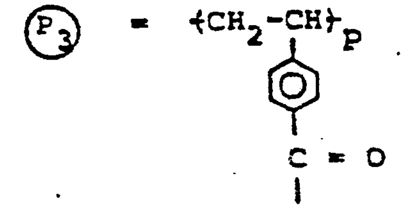

réticulée, en particulier avec du divinylbenzène.

12. Procédé selon l'une des revendications 1 à 11, caractérisé en ce que ladite résine polymère porte, en plus des chaînes latérales à groupement carbonylé, des groupes fonctionnels hydrophiles, tels que des groupements carboxyliques, amines primaire, secondaire, tertiaire ou ammoniums quaternaires.

13. Procédé selon l'une des revendications 1 à 12, caractérisé en ce que la capacité de ladite résine polymère en sites carbonyliques terminaux à activité catalytique est d'environ de 0,5 à 5 milliéquivalents par gramme de polymère.

14. Procédé selon l'une des revendications 1 à 13, caractérisé en ce que ladite résine polymère est introduite dans le milieu réactionnel à raison d'environ 10 g à environ 2 000 g de résine par mole d'α-amino-nitrile de départ.

15. Procédé selon l'une des revendications 1 à 13, caractérisé en ce que la solution d'α-amino-nitrile est mise au contact de ladite résine polymère immobilisée dans un réacteur, à raison d'environ 0,1 à environ 50 équivalents de composés carbonylés correspondants par mole d'α-amino-nitrile de départ.

16. Procédé selon l'une des revendications 1 à 15, caractérisé en ce que la température du milieu réactionnel est sensiblement comprise entre 5 et 80 °C.

17. Procédé selon l'une des revendications 1 à 16, caractérisé en ce que les ions hydroxydes sont introduits dans le milieu réactionnel sous forme d'un hydroxyde de métal alcalin ou alcalino-terreux ou encore d'hydroxyde d'ammonium.

18. Procédé selon l'une des revendications 1 à 17, caractérisé en ce que le milieu réactionnel aqueux comporte une teneur minimale en eau correspondant à 1 mole d'eau par mole d'α-amino-nitrile de départ.

19. Procédé selon l'une des revendications 1 à 18, caractérisé en ce que l'on réalise l'hydrolyse catalytique chimique en phase hétérogène d'un α-amino-nitrile répondant à la formule générale

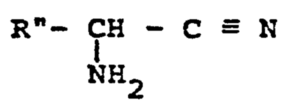

dans laquelle le radical R″ représente un atome d'hydrogène ou une chaîne hydrocarbonée, linéaire ou ramifiée, contenant de 1 à 12 atomes de carbone et éventuellement un ou plusieurs hétéroatomes de soufre, ledit radical R″ étant éventuellement substitué une ou plusieurs fois, de préférence en fin de chaîne, par des groupements hydroxy, amino, carboxyle, phényle, hydroxy-phényle, carboxamide, indolyle, iminazyle et guanidyle, ou bien R″ forme avec l'atome d'azote en position α un groupe hétérocyclique saturé contenant au moins un hétéroatome d'azote, ledit groupe hétérocyclique pouvant lui-même être substitué par un groupe hydroxy, ou de l'un de ses sels, tels que le chlorhydrate.

20. Procédé selon l'une des revendications 1 à 19, caractérisé en ce que le sel de l'α-amino-nitrile est le chlorhydrate.

21. Procédé selon l'une des revendications 1 à 20, caractérisé en ce que l'α-amino-nitrile est préparé par action d'un aldéhyde et d'acide cyanhydrique ou de cyanure, tel qu'un cyanure alcalin, sur de l'ammoniaque et un sel d'ammonium.

22. Procédé selon les revendications 20 et 21, caractérisé en ce que l'on laisse s'installer l'équilibre de formation de l'α-amino-nitrile avant d'effectuer sa mise en contact avec ladite résine polymère.

23. Procédé selon les revendications 20 et 21, caractérisé en ce que l'on stabilise la solution d'α-amino-nitrile par un excès de 5 à 10 % de cyanure par rapport au cyanure contenu dans la solution équimolaire aldéhyde-cyanure ou par rapport à la solution de cyanhydrine.

24. Utilisation pour l'hydrolyse catalytique chimique d'un alpha-amino-nitrile ou l'un de ses sels en vue d'obtenir l'amide ou l'acide correspondant, d'un catalyseur caractérisé en ce qu'il est constitué par une résine polymère, telle que définie dans l'une des revendications 1 à 13.

## Claims

1. Process for the chemical catalytic hydrolysis of an alpha-amino-nitrile or one of its salts in order to

produce the amide of the corresponding acid, characterized in this that the said alpha-amino-nitrile or one of its salts is made to react in an aqueous medium, in a heterogeneous phase and in the presence of hydroxide ions, on an insoluble polymer resin in a basic aqueous medium and in accordance with the following general formula :

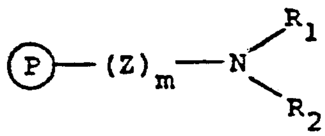

in which :

Ⓟ represents a matrix of polymer resin,

Z represents the formula group

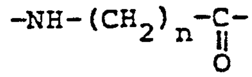

with $1 \leq n \leq 5$,

$m = 0$ or 1,

$R_1$ represents a hydrogen atom,

$R_2$ represents a cyclanone group with 4 to 7 chains comprising possibly 1 to 3 heteroatoms of nitrogen or a group of formula :

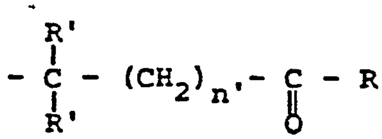

in which :

R' designates a hydrogen atom or a methyl or ethyl radical,

R designates a methyl or ethyl radical, and,

$n' = 0$ to 3,

or alternatively $R_1$ and $R_2$ together form with the adjacent nitrogen atom a cyclanone group of 5 to 7 chains comprising eventually 1 or 2 additional heteroatoms of nitrogen, and capable eventually of being substituted by one or more methyl or ethyl radicals, and in that in a manner known *per se*, hydroxide ions are introduced into the aqueous reaction medium at the rate of 0.1 to 0.3 mole per mole of alpha-amino-nitrile initially present in order to obtain the corresponding alpha-amino-amide, or alternatively hydroxide ions are introduced into the aqueous reaction medium in such a manner as to achieve substantially equimolarity with respect to the alpha-amino-nitrile initially used in order to obtain the corresponding alpha-amino-acid.

2. Process according to claim 1, characterized in that the said polymer resin is in acccordance with the general formula I

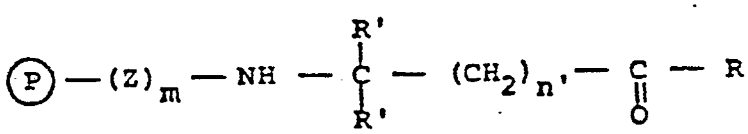

(I)

in which :

Ⓟ represents a polymer resin matrix,

Z represents the group of formula

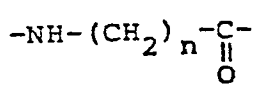

with $1 \leq n \leq 5$,

R' represents a hydrogen atom or a methyl or ethyl radical,

R represents a methyl or ethyl radical, and

$m = 0$ or 1

$n' = 0$ to 3

3. Process according to claim 1, characterized in that the said polymer resin is in accordance with the general formula II

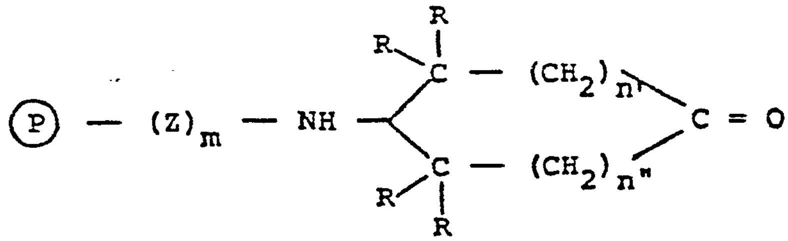 (II)

in which :

Ⓟ represents a polymer resin matrix,

Z represents a group of formula

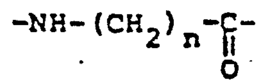

with $1 \leqslant n \leqslant 5$,

R represents a hydrogen atom or a methyl or ethyl radical,

m = 0 or 1,

n' = 0 to 3,

n" = 0 to 3,

n' + n" ⩽ 3.

4. Process according to claim 1, characterized in this that the said polymer resin is in accordance with the general formula III

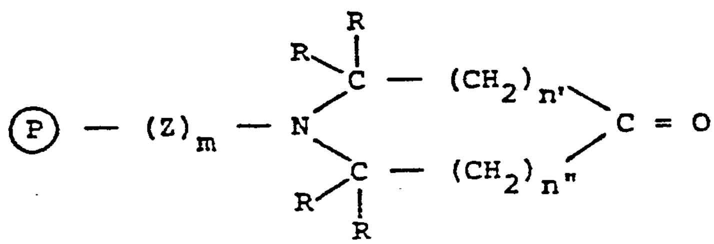 (III)

in which :

Ⓟ represents a polymer resin matrix,

Z represents the formula

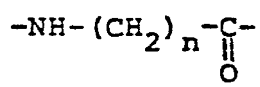

with $1 \leqslant n \leqslant 5$,

R represents a hydrogen atom or a methyl or ethyl radical,

m = 0 or 1,

n' = 0 to 3,

n" = 0 to 3,

n' + n" ⩽ 3.

5. Process according to claim 3, characterized in that the said polymer resin is in accordance with the general formula II in which the cyclanone group of formula

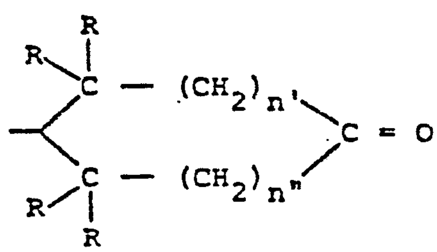

represents the radicals

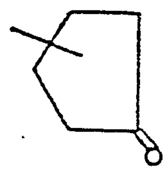, 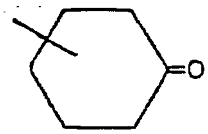 or 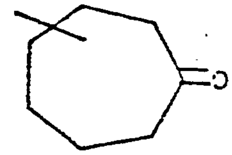

6. Process according to claim 4, characterized in that the said polymer resin is in accordance with the general formula III in which the heterocyclanone of formula

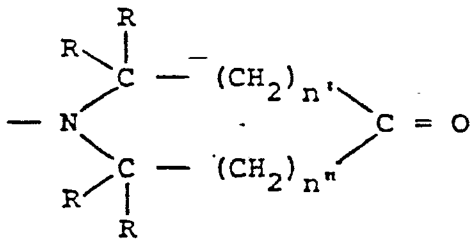

is selected amongst the radicals

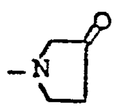 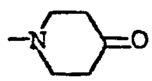 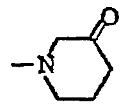 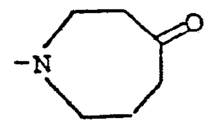 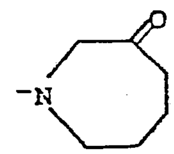

7. Process according to one of claims 1 to 6, characterized in this that the polymer resin matrix Ⓟ is of polystyrene, polyacrylic or cellulosic type.

8. Process according to claim 7, characterized in this that the polymer resin matrix Ⓟ is of the polystyrene or polyacrylic reticulated type.

9. Process according to claim 8, characterized in that the polymer resin matrix Ⓟ is a polyacrylic resin of formula

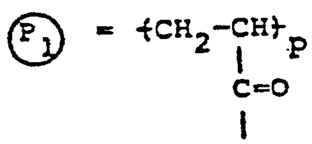

which is reticulated, in particular with divinylbenzene or with an N,N'bis acryloyl polymethylene diamine in accordance with the general formula (A) :

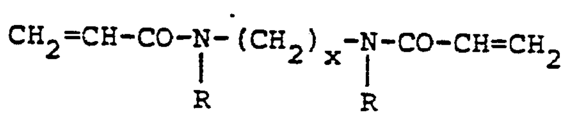 (A)

in which :

$x = 1$ to 6

R = H, $—CH_3$ or $—CH—_2$ $—CH_3$

where the two radicals R together form an alcoylene group of formula $(—CH_2—)_y$, and

$y = 1$ to 4.

10. Process according to claim 8, characterized in this that the polymer resin matrix Ⓟ is a polystyrene resin of formula

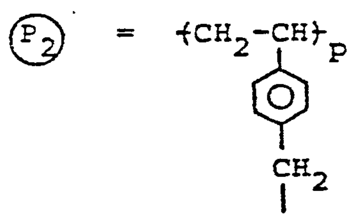

which is reticulated, in particular with divinylbenzene.

11. Process according to claim 8, characterized in that the polymer resin matrix Ⓟ is a resin of formula

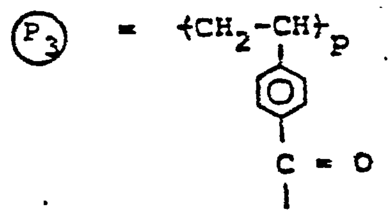

which is reticulated, in particular with divinylbenzene.

12. A process according to one of claims 1 to 11, characterized in that the said polymer resin carries, at several lateral chains with a carbonyl grouping, hydrophilic functional groups, such as carboxylic groups, primary, secondary or tertiary amines or quaternary ammonium radicals.

13. Process according to one of claims 1 to 12, characterized in this that the capacity of the said polymer resin at carbonyl terminal sites with catalytic activity is about 0.5 to 5 milliequivalents per gram of polymer.

14. Process according to one of claims 1 to 13, characterized in that the said polymer resin is introduced into the reaction medium at a rate of about 10 g to about 2 000 g of resin per mole of α-amino-nitrile as the starting material.

15. Process according to one of claims 1 to 13, characterized in this that the solution of α-amino-nitrile is brought into contact with the said immobilised polymer resin in a reactor, at a rate of about 0.1 to about 50 equivalents of corresponding carbonyl compounds per mole of α-amino-nitrile as the starting material.

16. Process according to one of claims 1 to 15, characterized in that the temperature of the reaction medium is substantially between 5 and 80 °C.

17. Process according to one of claims 1 to 16, characterized in this that the hydroxide ions are introduced into the reaction medium in the form of an alkaline metal or alkaline earth hydroxide or alternatively ammonium hydroxide.

18. Process according to one of claims 1 to 17 characterized in that the aqueous reaction medium comprises a minimum amount of water corresponding to 1 mole of water per mole of α-amino-nitrile as the starting material.

19. Process according to one of claims 1 to 18, characterized in that the catalytic chemical hydrolysis in a heterogeneous phase with an α-amino-nitrile is in accordance with the general formula

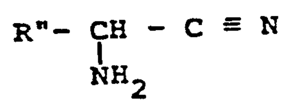

in which the radical R″ represents a hydrogen atom or a hydrocarbon chain, linear or branched, containing from 1 to 12 atoms of carbon and possibly one or several heteroatoms of sulphur, the said radical R″ being possibly substituted one or several times, preferably at the end of the chain, by hydroxy, amino, carboxyl, phenyl, hydroxyphenyl, carboxamide, indoyl, iminazyl and guanidyl, or alternatively R″ forms with the nitrogen atom in the position α, a saturated heterocyclic group containing at least one heteroatom of nitrogen, the said heterocyclic group being itself substituted by a hydroxy group, or one of its salts, such as chlorohydrate.

20. Process according to one of claims 1 to 19, characterized in this that the salt of the α-amino-nitrile is chlorohydrate.

21. Process according to one of claims 1 to 20, characterized in this that the α-amino-nitrile is prepared by the action of an aldehyde and cyanohydro acid or cyanide, such as an alkaline cyanide, on ammonia or an ammonium salt.

22. Process according to claims 20 and 21, characterized in this that equilibrium in the formation of α-amino-nitrile is permitted to take place before effecting the contacting with the said polymer resin.

23. Process according to claims 20 and 21, characterized in that the α-amino-nitrile is stabilised by an excess of 5 to 10 % of cyanide with respect to the cyanide contained in the equimolar aldehyde-cyanide or in relation to the cyanhydrin solution.

24. Use for the chemical catalytic hydrolysis of an α-amino-nitrile or one of its salts with the object of obtaining the amide or the corresponding acid, by a catalyser characterized in that it is constituted by a polymer resin, such as defined in one of claims 1 to 13.

**Patentansprüche**

1. Verfahren zur katalytischen chemischen Hydrolyse eines α-Aminonitrils oder eines Salzes

desselben zur Gewinnung des entsprechenden Amids bzw. der entsprechenden Säure, dadurch gekennzeichnet, daß man das α-Aminonitril oder dessen Salz in wäßrigem Milieu, in heterogener Phase und in Anwesenheit von Hydroxidionen auf einem in basischem wäßrigem Milieu unlöslichen Harzpolymeren der folgenden allgemeinen Formel :

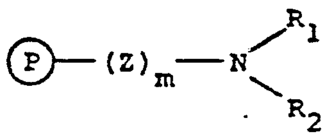

worin bedeuten :

Ⓟ eine Matrix des Harzpolymeren ;

Z eine Gruppe der Formel

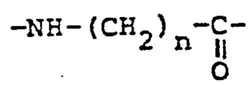

mit 1 ≤ n ≤ 5 ;

m = 0 oder 1 ;

$R_1$ ein Wasserstoffatom ;

$R_2$ eine 4- bis 7-gliedrige und gegebenenfalls 1 bis 3 Stickstoffheteroatom(e) enthaltende cyclische Ketongruppe oder eine Gruppe der Formel :

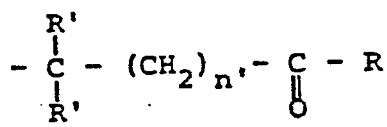

in welcher darstellen :

R′ ein Wasserstoffatom oder eine Methyl- oder Ethylgruppe ;

R eine Methyl- oder Ethylgruppe und

n′ = 0-3,

oder $R_1$ und $R_2$ zusammen mit dem benachbarten Stickstoffatom eine gegebenenfalls 1 oder 2 zusätzliche(s) Stickstoffheteroatom(e) enthaltende und gegebenenfalls durch eine oder mehrere Methyl- oder Ethylgruppe(n) substituierte, 5- bis 7-gliedrige cyclische Ketongruppe, reagieren läßt und in an sich bekannter Weise zur Gewinnung des entsprechenden α-Aminoamids, bezogen auf 1 Mol Ausgangs-α-Aminonitril 0,1-0,3 Mol Hydroxidionen in das wäßrige Reaktionsmilieu einführt oder zur Gewinnung der entsprechenden α-Aminosäure eine solche Menge an Hydroxidionen in das wäßrige Reaktionsmilieu einführt, daß man in bezug auf das Ausgangs-α-Aminonitril praktisch eine Äquimolarität erreicht.

2. Verfahren nach Anspruch 1, dadurch gekennzeichnet, daß das Harzpolymere der allgemeinen Formel :

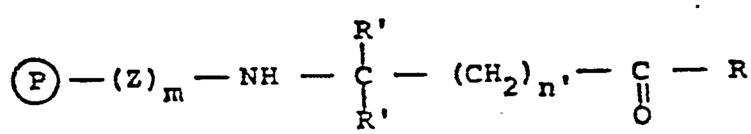 (I)

worin bedeuten :

Ⓟ eine Matrix des Harzpolymeren ;

Z eine Gruppe der Formel

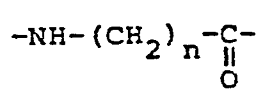

mit 1 ≤ n ≤ 5 ;

R′ ein Wasserstoffatom oder eine Methyl- oder Ethylgruppe ;

R ein Wasserstoffatom oder eine Methyl- oder Ethylgruppe ;

m = 0 oder 1 und

n′ = 0-3,

entspricht.

3. Verfahren nach Anspruch 1, dadurch gekennzeichnet, daß das Harzpolymere der allgemeinen Formel :

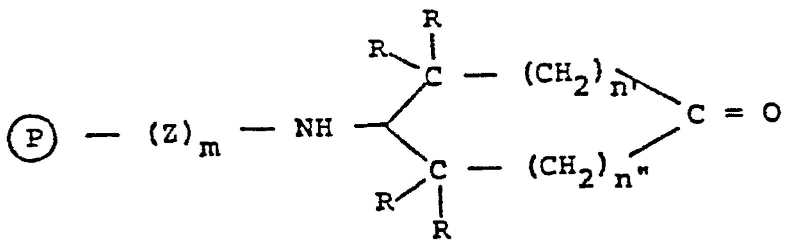 (II)

worin bedeuten :

Ⓟ eine Matrix des Harzpolymeren ;

Z eine Gruppe der Formel

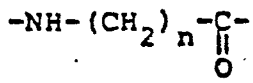

mit $1 \leqslant n \leqslant 5$ ;

R ein Wasserstoffatom oder eine Methyl- oder Ethylgruppe ;

m = 0 oder 1 ;

n' = 0-3 und

n" = 0-3, wobei gilt, daß n' + n" < 3

entspricht.

4. Verfahren nach Anspruch 1, dadurch gekennzeichnet, daß das Harzpolymere der allgemeinen Formel :

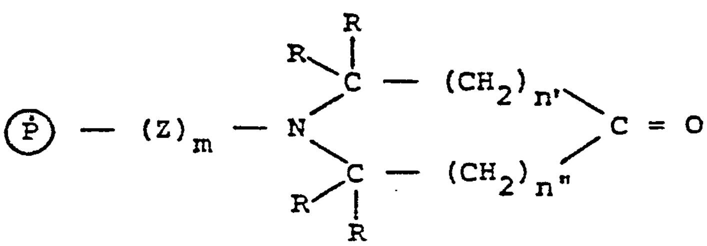 (III)

worin bedeuten :

Ⓟ eine Matrix des Harzpolymeren ;

Z eine Gruppe der Formel

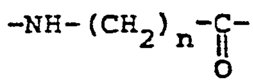

mit $1 \leqslant n \leqslant 5$ ;

R ein Wasserstoffatom oder eine Methyl- oder Ethylgruppe ;

m = 0 oder 1 ;

n' = 0-3 und

n" = 0-3, wobei gilt, daß n' + n" < 3

entspricht.

5. Verfahren nach Anspruch 3, dadurch gekennzeichnet, daß das Harzpolymere der allgemeinen Formel (II) entspricht, worin die cyclische Ketongruppe der Formel :

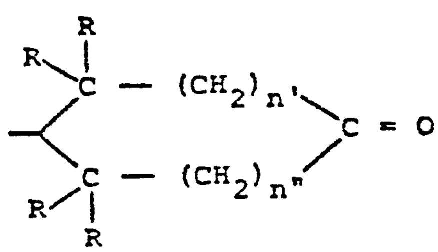

durch die Reste :

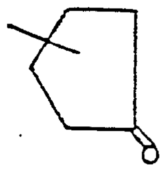,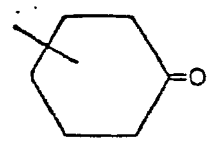 oder 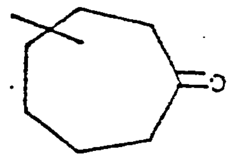

darstellbar ist.

6. Verfahren nach Anspruch 4, dadurch gekennzeichnet, daß das Harzpolymere der allgemeinen Formel (III) entspricht, wobei die heterocyclische Ketongruppe der Formel :

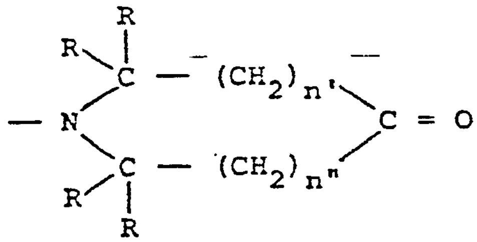

aus den Resten :

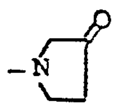 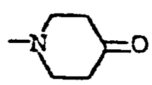 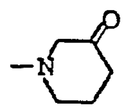 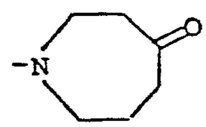 oder 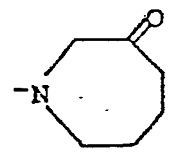

ausgewählt ist.

7. Verfahren nach einem der Ansprüche 1 bis 6, dadurch gekennzeichnet, daß die Matrix des Harzpolymeren Ⓟ vom Polystyrol-, Polyacryl- oder Cellulosetyp ist.

8. Verfahren nach Anspruch 7, dadurch gekennzeichnet, daß die Matrix des Harzpolymeren Ⓟ vom vernetzten Polystyrol- oder Polyacryltyp ist.

9. Verfahren nach Anspruch 8, dadurch gekennzeichnet, daß die Matrix des Harzpolymeren Ⓟ aus einem Polyacrylharz der Formel

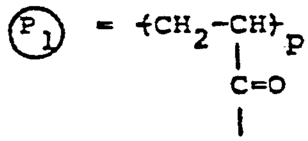

das insbesondere mit Divinylbenzol oder mit einem N,N'-Bis(acryloylpolymethylendiamin) der allgemeinen Formel :

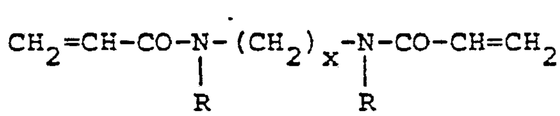 (A)

worin

x = 1-6,

R = H, $—CH_3$ oder $CH_2—CH_3$ oder die beiden Reste R zusammen eine Alkylengruppe der Formel $(—CH_2—)_y$ mit y = 1-4 bilden,

vernetzt ist, besteht.

10. Verfahren nach Anspruch 8, dadurch gekennzeichnet, daß die Matrix des Harzpolymeren Ⓟ aus einem Polystyrolharz der Formel :

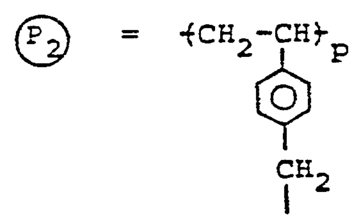

das insbesondere mit Divinylbenzol vernetzt ist, besteht.

11. Verfahren nach Anspruch 8, dadurch gekennzeichnet, daß die Matrix des Harzpolymeren Ⓟ aus einem Harz der Formel :

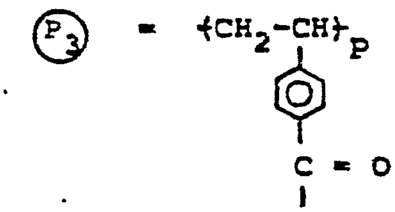

das insbesondere mit Divinylbenzol vernetzt ist, besteht.

12. Verfahren nach einem der Ansprüche 1 bis 11, dadurch gekennzeichnet, daß das Harzpolymere neben den Seitenketten mit carbonylierter Gruppe funktionelle hydrophile Gruppen, wie Carboxyl-, primäre, sekundäre oder tertiäre Amin- oder quaternäre Ammoniumgruppen trägt.

13. Verfahren nach einem der Ansprüche 1 bis 12, dadurch gekennzeichnet, daß das Harzpolymere in den endständigen Carbonylstellen eine Kapazität an katalytischer Aktivität von etwa 0,5 bis 5 mÄq/g Polymeres besitzt.

14. Verfahren nach einem der Ansprüche 1 bis 13, dadurch gekennzeichnet, daß das Harzpolymere in das Reaktionsmilieu im Verhältnis von etwa 10 g bis etwa 2 000 g Harz pro Mol Ausgangs-α-Aminonitril eingeführt wird.

15. Verfahren nach einem der Ansprüche 1 bis 13, dadurch gekennzeichnet, daß man die Lösung des α-Aminonitrils mit dem immobilisierten Harzpolymeren in einem Reaktor im Verhältnis von etwa 0,1 bis etwa 50 Äquivalent(e) an den entsprechenden carbonylierten Verbindungen pro Mol Ausgangs-α-Aminonitril in Kontakt bringt.

16. Verfahren nach einem der Ansprüche 1 bis 15, dadurch gekennzeichnet, daß die Temperatur des Reaktionsmilieus im wesentlichen von 5-80 °C reicht.

17. Verfahren nach einem der Ansprüche 1 bis 16, dadurch gekennzeichnet, daß man die Hydroxidionen in das Reaktionsmilieu in Form von Alkalimetall- oder Erdalkalimetall- oder auch Ammoniumhydroxiden einführt.

18. Verfahren nach einem der Ansprüche 1 bis 17, dadurch gekennzeichnet, daß das wäßrige Reaktionsmilieu einen Mindestwassergehalt entsprechend 1 Mol Wasser/Mol Ausgangs-α-Aminonitril enthält.

19. Verfahren nach einem der Ansprüche 1 bis 18, dadurch gekennzeichnet, daß man bei der in heterogener Phase durchgeführten katalytischen chemischen Hydrolyse ein α-Aminonitril der allgemeinen Formel

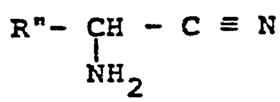

worin R″ für ein Wasserstoffatom oder eine lineare oder verzweigte, 1-12 Kohlenstoffatom(e) sowie gegebenenfalls ein oder mehrere Schwefelheteroatom(e) enthaltende Kohlenwasserstoffkette steht, wobei der Rest R″ gegebenenfalls ein- oder mehrfach, vorzugsweise am Kettenende, durch Hydroxy-, Amino-, Carboxyl-, Phenyl-, Hydroxyphenyl-, Carboxamid-, Indolyl-, Iminazyl- und Guanidylgruppen substituiert sein kann, oder der Rest R″ zusammen mit dem Stickstoffatom in α-Stellung eine mindestens ein Stickstoffheteroatom enthaltende heterocyclische Gruppe, die ihrerseits durch eine Hydroxygruppe substituiert sein kann, bilden kann, oder ein Salz, z. B. Hydrochlorid, desselben hydrolysiert.

20. Verfahren nach einem der Ansprüche 1-19, dadurch gekennzeichnet, daß man als α-Aminonitrilsalz dessen Hydrochlorid einsetzt.

21. Verfahren nach einem der Ansprüche 1-20, dadurch gekennzeichnet, daß man das α-Aminonitril durch Einwirkung eines Aldehyds und von Cyanwasserstoffsäure oder eines Cyanids, z. B. eines Alkalimetallcyanids, auf Ammoniak oder ein Ammoniumsalz herstellt.

22. Verfahren nach Ansprüchen 20 und 21, dadurch gekennzeichnet, daß man sich das Bildungsgleichgewicht des α-Aminonitrils einstellen läßt, bevor man es mit dem Harzpolymeren in Kontakt bringt.

23. Verfahren nach Ansprüchen 20 und 21, dadurch gekennzeichnet, daß man die Lösung des α-Aminonitrils mit einem 5- bis 10 %igen Cyanidüberschuß in bezug auf den Cyanidgehalt in der äquimolaren Aldehyd/Cyanid-Lösung oder in bezug auf die Cyanhydrinlösung stabilisiert.

24. Verwendung eines gemäß einem der Ansprüche 1 bis 13 hergestellten Harzpolymeren als Katalysator bei der katalytischen chemischen Hydrolyse eines α-Aminonitrils oder Salzes desselben zur Gewinnung des entsprechenden Amids bzw. der entsprechenden Säure.